Europäisches Patentamt

European Patent Office

Office européen des brevets

(11) Publication number: **0 123 426**
**B1**

## EUROPEAN PATENT SPECIFICATION

(45) Date of publication of patent specification: **19.10.88**

(51) Int. Cl.⁴: **A 61 F 2/00, B 32 B 27/12**

(21) Application number: **84301919.1**

(22) Date of filing: **21.03.84**

(54) Biocompatible composite material for implants and artificial organs for the human body.

(30) Priority: 23.03.83 JP 48293/83
07.02.84 JP 20380/84

(43) Date of publication of application:
31.10.84 Bulletin 84/44

(45) Publication of the grant of the patent:
19.10.88 Bulletin 88/42

(84) Designated Contracting States:
DE FR GB IT

(56) References cited:
DE-A-2 910 627
GB-A-1 165 698
GB-A-2 092 891

(73) Proprietor: KUREHA KAGAKU KOGYO
KABUSHIKI KAISHA
9-11 Horidome-cho 1-chome Nihonbashi Chuo-
ku
Tokyo 103 (JP)

(72) Inventor: Takabe, Reiho
1585 Shukugahara Tama-ku
Kawasaki-shi Kanagawa-ken (JP)

(74) Representative: Myerscough, Philip Boyd et al
J.A.Kemp & Co. 14, South Square Gray's Inn
London, WC1R 5EU (GB)

# 0 123 426

**Description**

The present invention relates to composite materials possessing excellent biocompatibility and useful as a material for implants and artificial organs for the body.

Hitherto, various kinds of biocompatible materials have contributed to the development of techniques in artificial organs. For instance, carbon materials, particularly graphite obtained by thermal composition of carbonaceous materials, have been broadly used as a material compatible with the living body, and the biocompatibility therefor has been well recognized.

However, accompanying the development of various techniques of artificial organs, the application of conventional biocompatible materials to new fields or the development of a new biocompatible material, which is provided with biocompatibility suitable for specified objectives and a specified device, has been demanded. For instance, it has been desired to develop a flexible material of which the outer side has excellent biocompatibility (compatibility with the tissues) and of which the inner side is thrombo-resistant (compatibility with blood). It has also been desired to make the excellent biocompatible carbonaceous material or ceramic material, which are only used as artificial hard material for artificial bones and artificial tooth roots, usable as artificial soft material with biocompatibility.

GB—A—1165698 is concerned with prostheses made, at least substantially, from elemental carbon or graphite. These prostheses are made into the form of a non-metallic, electrically non-insulating artificial member or piece of tissue for the body. Amongst the Figures, Figure 7 illustrates a carbon tube provided with an outer plastics liner to compensate for possible weaknesses in prostheses formed from the carbon tube alone. The carbon tube and plastics material are adhered together only at their ends.

GB—A—2092891 relates to a prosthetic material for tooth or bone loss. The material comprises a substrate made of a carbon or metal which is corrosion resistant and has high afinity to vital tissue and a porous surface layer of carbon. This material must be hard, not flexible.

According to the present invention, there is provided a flexible biocompatible composite material suitable for use in medical devices, comprising a layer of aggregate fiber material having a layer of plastics material secured thereto, wherein the opposed surfaces of the said layers are bonded together throughout and the said layer of aggregate fiber material is arranged for penetration by tissue cells. The composite material can be shaped. The material may be used for implants and artificial organs for the human body.

In the description which follows, reference will be made to the accompanying drawing in which the Figure is a schematic diagram of apparatus for carrying out chemical vapour deposition (pyrolitic carbon deposition) on a fibrous material.

The present invention relates to a flexible composite material comprising a layer of an aggregate fiber material and a layer of flexible plastic material. In more detail, the composite material according to the present invention comprises a layer of aggregate fiber material, which has plenty of open pace of a relatively large average diameter resulting in allowing the entrance of tissue cells, and a layer of a biocompatible plastics material. The layers are bonded together and arranged as indicated above.

By applying the fiber-form of hard material such as carbon or ceramic material, the present invention has made the application of thus hard material to the biocompatible soft material. Thus, the flexible composite material according to the present invention has an excellent biocompatibility. Accordingly, it is applicable to various artificial organs and is useful as, for instance, the material for implants applied via the skin (percutaneous system), the implant being applied through the skin for taking various substances in and out from a living body, such as the device for blood access used in artificial hemodialysis, and the device for transmitting electricity and motive force for driving the embedded-type artificial organs.

The aggregate fiber material according to the present invention is typically a primary-processed product and is exemplified by, for instance, products obtained by knitting and/or braiding fibers into cloth, non-woven cloth or felt. The form and shape of the aggregate fiber material may be selected according to the objective and application of the composite material. It is preferable that the aggregate fiber material has openings or free stomata of 20 to 1,000 μ.

As the fibrous material constructing the aggregate fiber material, biocompatible heat-resistant fibers, for instance, inorganic fibers such as carbon fibers, graphite fibers, glass fibers, silica fibers, zirconia fibers or apatite fibers and material fibers such as stainless steel fibers, titanium fibers, fibers of alumina or fibers of boron may be exemplified. These fibers are used singly or as a composite. Further, the shape, form and diameter of these fibrous material are not limited, and as the shape, monofilament, multifilament, twisted thread, spun yarn, staple fiber and whisker may be exemplified.

The fibrous material is used as it is or after being coated with carbon by the chemical vapour deposition method (hereinafter referred to as CVD). In particular, the inorganic fibers and metal fibers are preferably coated with carbon from the viewpoint of improving biocompatibility. The CVD method comprises the steps of pyrolysis of a gaseous hydrocarbon, for instance, methane, ethylene, propane, butane, benzene or toluene at a temperature of higher than the decomposition temperature thereof and depositing the thus formed carbon the fibers to cover them. The temperature used for the hydrocarbon-decomposition step is generally 600 to 3,000°C, preferably 700 to 2,500°C.

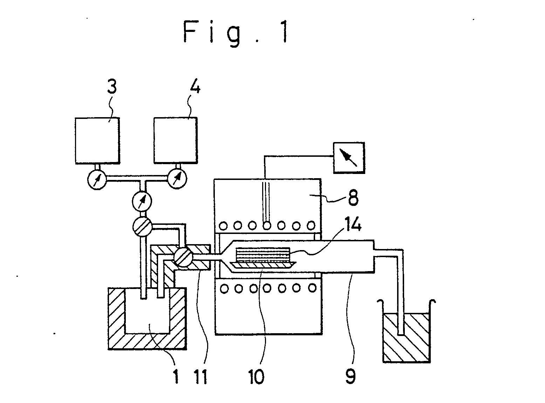

Figure 1 shows an example of the apparatus used for coating the aggregate fiber material with carbon by the CVD method, and in Figure 1, *14* is the aggregate fiber material to be treated by CVD method; *1* is a gas-trap; *10* is a quartz plate and *11* is a preheating zone. In CVD, an inert gas *3* such as argon, hydrogen

2

and nitrogen is used as a carrier and a hydrocarbon *4* is introduced into a quartz tube *9* placed in an electric furnace *8* kept at a temperature of 600 to 3,000°C, preferably 700 to 2,500°C. The carbon coating is carried out, in general, for 5 to 180 min.

The thus obtained, coated aggregate fiber material may be optionally subjected to thermal treatment in an inert gaseous atmosphere at temperature of higher than that of the CVD method.

Although the treatment by the CVD method may be carried out on the fiber form itself, it is preferably carried out on the processed products such as knit and/or braid bodies, cloth, non-woven cloth, felt and wound yarn.

The flexible plastics material used according to the present invention is prepared by methods such as injection molding, extruding, pressing, vacuum molding and the like, or the products obtained by further processing thereof, and the shape thereof may be exemplified by column, tube, sheet, film and complicated three-dimensional structure. The shape may be selected according to the objective and position of application of the composite material according to the present invention, and the tube-form shaped material has a broad field of utilization as will be described later.

The biocompatible plastics material employed according to the present invention includes elastomer, and may be any plastics so far as it is a generally commercialized biocompatible plastic material. For instance, fluoropolymer such as polytetrafluoroethylene and polyvinylidene fluoride, silicone resin such as silicone rubber, copolymer of vinylidene chloride, polyvinyl chloride, polyethylene, polypropylene, polyester, poly(hydroxymethyl methacrylate), polyacrylamide, polysulfone, poly(N-vinylpyrrolidone) and segmented polyurethane may be exemplified.

It is also preferable to subject these plastics materials to surface-treatment by etching, glow-discharging or coating with a surface-treating agent for improving the adhesion thereof as will be shown later. Further, it is effective to coat the contact surface of the shaped plastics material, which is contacted directly by blood, with an anti-blood coagulant such as heparin, urokinase, albumin and streptokinase.

The joining of the layer of the aggregate fiber material and the layer of the plastics material may be effected by any desired method so far as the porosity and flexibility of the layer of the aggregate fiber material are not impaired. Most generally, an adhesive agent is painted on one surface of the layer of the plastics material as a thin film, and a layer of the aggregate fiber material is piled on the painted layer by applying a pressure. Instead of using the adhesive agent, the bonding surface of the layer of the plastics material is brought into molten state, and then the layer of the aggregate fiber material is piled thereon, thereby joining the two layers. A method may be taken in which the fibrous material is directly, wound or planted on to the surface of the layer of the plastics material, on which the adhesive agent has been painted or which is partially molten.

Furthermore, since the fibrous material used herein does not substantially expand nor contract, in the case of joining it is necessary to be careful to adopt a method of joining by which a stress is not loaded in the direction of fiber axis but loaded in the direction of bending. For instance, since the yarn used for preparing cloth has been twisted, the position of one filament of the thus twisted yarn in the cross-section of the twisted yarn is different in every cross-section of the twisted yarn. Accordingly, in the case where the thickness of the layer of the adhesive agent used in the joining is less than the radius of the twisted yarn, the probability of adhesion of the single filament is less than 50%. So the remaining part of the single filament is not adhered, the remaining part thereof may be freely bent. This situation appears as the flexibility of the layer of aggregate fiber material (in this case, a cloth) joined to the layer of plastics material. Consequently, in order to obtain a sufficient flexibility for practical use, it is preferable that the thickness of the layer of the adhesive agent is less than a quarter of the thickness of the layer of the aggregate fiber material.

As the adhesive agent for use in the joining, a silicone, copolymer of ethylene and vinyl acetate, polyester, nylon, urethane-elastomer, vinyl acetate resin and acrylic resin may be exemplified.

The presence of open spaces formed between the single filaments and between the twisted-yarns without filling of the adhesive agent is necessary for providing the layer of the aggregate fiber material having the flexibility and allowing the tissue cells to be infiltrated and to be solidified therein.

Further, in the case where a sufficient strength is available, a mechanical power may be utilized by winding the fibrous materials on the plastic material or by covering the plastics material with a knit bag-type fibrous material and fixing the bag thereon.

The composite material according to the present invention may be not only composed of the two layers prepared by joining the layer of the aggregate fiber material and the layer of the plastics material but also composed of the three layers of a construction that a layer of the plastics material as a core layer is disposed between two layers of the aggregate biocompatible fiber material on both sides of the core layer.

Furthermore, the composite material comprising the layer of the plastics material in which an electroconductive material such as copper wire has been incorporated and a layer of the aggregate fiber material is extremely useful in the field where it is necessary to take out informations through living body as electrical signals.

The thus obtained composite material according to the present invention is a flexible material comprising a remarkably porous layer of the aggregate biocompatible fiber material and a layer of shaped biocompatible plastics material and is usable in various purposes. As an example of the various uses, a use as the implant material applied via the skin (percutaneously) may be exemplified.

**0 123 426**

More concretely, the composite material according to the present invention is applied to the techniques of artificial organs, which have the objectives of taking in and out blood in cases of hemodialysis, hemoperfusion, hemofiltration, plasmaexchange, plasmafiltration and peritoneal dialysis, taking in and out of a heating liquid in the case of heating the internal organs (hyperthermia), introducing a lead-in wire for supplying electricity for driving an artificial heart or artificial pancreas, introducing a heating device and/or temperature sensor in hyperthermia therapy, and taking out of electrical signals of the information in living body such as the topical temperature and electromotive force within living body. In every one of the above-mentioned cases, it is necessary to take in and out some substances through the skin of a living body (percutaneously), and in such a case, a material which cannot fulfill both the objectives of taking in and out substances through the skin and of joining the material with the skin cannot be used because of the fear of infection due to the incompleteness of joining to the skin with the material at the position where the material penetrates the skin. On the other hand, in the case of the composite material according to the present invention, for instance, the composite material comprises a plastics tube covered with a knit bag made of carbon fiber, the tissue of the skin penetrates into the layer of the aggregate fiber material and clings thereto as it is, and as a result, the thus applied composite material can be used for a long period of time without causing any infection. Further, by utilizing the specific property of the composite material according to the present invention of easily allowing the penetration and rooting of the cells into the aggregate fiber material, the composite material can be utilized as a substrate for culturing the various cells and tissues. In such a case, the shape of the composite material is preferably sheet form or film form.

The following Examples illustrate the present invention.

Example 1
Preparation of a tube-form composite material

After coating a silicone tube of 3.3 mm in inner diameter, 4.6 mm in outer diameter and 30 mm in length with a silicone adhesive at a thickness of 0.1 to 0.3 mm, the thus coated tube was joined to a cloth (bag-net form) of an aggregate material of carbon fiber while pulling the cloth into both directions along the tube to obtain a tube-form composite material which retained the original softness.

For subjecting the thus prepared product to test, after closely sealing one of the ends thereof, the tube was cut in length of 10 mm. Separately, as an experimental animal, a SD rat of about 260 g in weight was incised on its back after shaving the back and under a light anesthesia by ether. The piece of the composite material sterilized with steam was implanted into the incised position while placing the closely sealed end in the body of the animal and the other end out of the body thereof, namely, the test piece was implanted through the skin of the animal. After one week of the operation, the vulnus has been cured and the tube has been fixed. Even after 2 months of the operation, the planted state of the tube was maintained as it was without any infection in the planted position. The animal was slaughtered thereafter to observe the interface of the skin and the implanted composite material. It was recognized that the cells of the skin penetrated into the layer of aggregate fiber material.

Comparative Example

A test was carried out in the same procedures as in Example 1 except for using only the silicone rubber tube instead of using the composite material in Example 1. The skin around the implanted position showed redness confirming the infection. The implanted tube was removed from the skin after about 2 weeks of the operation.

Example 2

After treating each of the cloth (bag-net form) respectively prepared by silica fiber, stainless steel fiber and graphite fiber by the CVD treatment instead of the cloth prepared by carbon fiber of Example 1, each of the silicone rubber tubes was joined to each of the three kinds of cloths in the same procedures as in Example 1. By respectively using the thus prepared three kinds of the composite material according to the present invention, the same tests as in Example 1 were carried out to observe the biocompatibility of the composite material.

The results were almost the same as in Example 1, namely, the skin penetrated into the layer of aggregate fiber material without causing infection.

Example 3

After painting an adhesive agent derived from polyvinyl acetate on the outer side of a copper wire covered with soft polyvinyl chloride, the thus painted copper wire of about 3 mm in outer diameter was joined to a cloth (bag-net form) made of silica fibers carbon-coated by the CVD treatment while pulling the cloth in both directions along the length of the wire. After closely sealing one of the ends of the thus covered wire with a layer of the adhesive agent, the wire was cut in 10 mm in length and the thus cut piece was implanted on the back of a SD rate while placing the seal end in the rat's body in the same manner as in Example 1. After about one week of the operation, the wound around the implanted position was cured, and even after one month of the operation, no infection was recognized with the fixed implanted material through the skin.

4

# 0 123 426

Example 4

On a flat plate, a sheet of polyvinyl chloride of 100 mm in length, 100 mm in width and 1 mm in thickness was placed, and two layers of a cellophane tape were sticked on all four sides thereof to provide a step of about 0.1 mm in height. Silastic® Silicone type A (an adhesive agent of a silicon resin) was poured into the thus prepared space made by the step of cellophane tapes, and a layer of the adhesive agent of about 0.1 mm in thickness was prepared by squeezing the poured adhesive agent with a glass rod.

After treating a cloth made of carbon fibers by the CVD method, the thus treated cloth was piled on the layer of the adhesive agent applied on the sheet of polyvinyl chloride, and after applying a pressure, the thus laminated material was left as it was for 24 hours to obtain a composite material composed of a sheet of polyvinyl chloride and a CVD-treated cloth of carbon fibers.

In the same procedure as above except for using an ion-etched sheet of polytetrafluoroethylene or a sheet of silicone rubber instead of the sheet of polyvinyl chloride, two kinds of the composite materials according to the present invention were prepared.

Further, as a comparative specimen, another composite material was prepared by piling a cloth of carbon fibers not treated by the CVD method on a sheet of silicone rubber.

The thus prepared composite materials respectively showed sufficient softness.

For use in the following test of determining the biocompatibility of each of the thus prepared composite materials, four circular specimens of 32 mm in diameter were cut out from each of the composite materials.

Each specimen of the composite material was placed in a glass dish of 42 mm in diameter, and after sterilizing by a steam gas, 5 ml of an aqueous suspension of $2 \times 10^4$ to $5 \times 10^4$ cells/ml was added respectively, and then the cells were cultured for 4 days at 37°C in an atmosphere containing 5% by volume of carbon dioxide. Thereafter, the cells grown on the composite material were removed off by treatment with trypsin and the number of the removed cells was calculated while using a blood corpuscle-calculating plate. Separately as the reference, the number of the cells grown on a plastics dish (made by Lux Company) under the same conditions as above was calculated.

Since the biocompatibility of the composite material corresponds to the rate of proliferation of the cells on the composite material, the rate of proliferation of the cells was calculated according to the following formula:

$$\text{Rate of proliferation} = \frac{A_t - A_o}{S_t - S_o}$$

wherein $A_o$ is the concentration of the cells at the start of culture; $A_t$ is the concentration of the cells after 4 days of culture; $S_o$ is the concentration of the cells at the start of culture on the plastics sheet (Lux Company) and $S_t$ is the concentration of the cells after 4 days of culture on the plastic sheet. The culture test was carried out 4 times to obtain the average rate of proliferation. The results are shown below.

5

**0 123 426**

TABLE
Rate of proliferation

Unit: %

| No. of specimen | Specimen | Cell[4] | |
|---|---|---|---|
| | | Ca.9.22 | RTG |
| 1 | Layer of PVC[1] only | 5 | 0 |
| 2 | Layer of PVC+Layer of CVD cloth[2] | 75 | 71 |
| 3 | Layer of polytetrafluoroethylene only | 53 | 50 |
| 4 | Layer of polytetrafluoroethylene+ Layer of CVD cloth | 78 | 75 |
| 5 | Layer of silicone rubber only | 55 | 50 |
| 6 | Layer of silicone rubber+Layer of CVD cloth | 80 | 78 |
| 7 | Layer of silicone rubber+Layer of cloth[3] | 75 | 73 |

Notes:
[1] Polyvinyl chloride.
[2] CVD-treated cloth of carbon fibers.
[3] Cloth of carbon fibers not treated by the CVD method.
[4] Cell Ca.9.22: Strained epitherial cell derived from human gingival cancer.
RTG: Fibroblast derived from rat foetal dental germ.

In Table, the specimens Nos. 1, 3 and 5 were not the composite materials.

As are seen in Table, the rate of proliferation was larger in the composite material than in the component (the plastics material), even in the case where the cloth of carbon fibers was not treated by the CVD method.

Example 5

After placing a sheet of polyvinyl chloride of 10 cm in width, 10 cm in length and 1 mm in thickness on a flat plate and painting the upper side of the sheet with an adhesive agent derived from silicone, a cloth made of glass fibers was placed on the thus painted side of the sheet and by applying a pressure to the laminate to obtain a composite material consisting of a layer of polyvinyl chloride and a layer of aggregate fiber material (cloth of glass fibers).

By using the thus prepared composite material, a test was carried out to obtain the rate of proliferation of the same epitherial cells as in Example 4. The rate of proliferation was 65%.

Example 6
Treatment of a cloth made of carbon fibers by CVD method

A cloth of carbon fibers was treated in an apparatus shown schematically in Fig. 1 by the CVD method.

After placing a cloth (14) made of carbon fibers on a quartz plate (10) placed in a quartz tube (9) of 55 mm in inner diameter and 30 cm in length of a uniform heating zone placed in an electric furnace, argon gas (3) was introduced at a rate of 100 ml/min into the quartz tube via a trap (1) after being pre-heated to 500°C by a ribbon heater (11) with the beginning of heating the electric furnace. From the time at which the temperature of the electric furnace became 1,000°C, methane gas (4) was also introduced into the furnace at a rate of 1 ml/min via the trap (1) after being preheated to 500°C. After introducing the gaseous mixture of argon and methane for about one hour, the supply of methane and electricity to the furnace and the preheater was stopped and thereafter, the apparatus and the specimen were cooled by the flow of argon gas. The thus CVD-treated cloth of carbon fibers was further thermally treated in a flow of argon at 2,000°C for 30 min. After cooling the thus treated specimen, a product of the cloth of carbon fibers treated by the CVD method was obtained, the product being referred to as PG cloth of carbon fibers.

**Claims**

1. A flexible biocompatible composite material comprising a layer of aggregate fiber material having a

6

**0 123 426**

layer of plastics material secured thereto, characterised in that the opposed surfaces of the said layers are bonded together throughout and the said layer of aggregate fiber material is arranged for penetration by tissue cells.

2. A material according to claim 1, wherein said aggregate fiber material is a primary processed product.

3. A material according to claim 2, wherein said primary processed product has been obtained by knitting and/or braiding fibers into cloth, non-woven cloth or felt.

4. A material according to any one of the preceding claims wherein said aggregate fiber material is made of biocompatible heat-resistant fiber.

5. A material according to claim 4, wherein said biocompatible heat-resistant fiber is inorganic or metal fiber.

6. A material according to claim 5, wherein said inorganic fiber is carbon fiber.

7. A material according to claim 4, wherein said biocompatible heat-resistant fiber is a carbon-coated fiber.

8. A material according to any one of the preceding claims, wherein said aggregate fiber material has openings or free stomata of 20 to 1,000 μm in diameter.

9. A material according to any one of the preceding claims, wherein said plastics material is a flexible biocompatible plastics material.

10. A material according to claim 9, wherein said flexible plastics material is a fluoropolymer, silicone resin, polyvinyl chloride, copolymer of vinylidene chloride, polyethylene, polypropylene, polyester, poly(hydroxymethyl methacrylate), polyacrylamide, polysulfone, poly(N-vinylpyrrolidone) or segmented polyurethane.

11. A material according to any one of the preceding claims, comprising a said layer of plastics material to each side of which is bonded a respective said layer of aggregate fiber material.

12. A material according to any one of the preceding claims, wherein the layers are bonded together by means of a layer of an adhesive agent interposed between said layer of aggregate fiber material and said layer of plastics material.

13. A material according to claim 12, wherein a said adhesive agent is an adhesive agent derived from a silicone, copolymer of ethylene and vinyl acetate, polyester, nylon, urethane-elastomer, vinyl acetate resin or acrylic resin.

14. A material according to any one of the preceding claims wherein said plastics material has an electroconducting material incorporated therein.

15. An implant or artificial organ for the human body, said implant or artificial organ being made of a flexible biocompatible composite material as claimed in any one of the preceding claims.

**Patentansprüche**

1. Flexibles, biokompatibles, zusammengesetztes Material, umfassend eine Schicht aus Aggregatfasermaterial mit einer daran befestigten Schicht aus Kunststoffmaterial, dadurch gekennzeichnet, daß die gegenüberliegenden Oberflächen der Schichten überall miteinander verbunden sind und die Schicht aus Aggregatfasermaterial für die Penetration durch Gewebezellen angeordnet bzw. eingerichtet ist.

2. Material nach Anspruch 1, wobei das Aggregatfasermaterial ein primär verarbeitetes Produkt ist.

3. Material nach Anspruch 2, wobei das primär verarbeitete Produkt erhalten wurde durch Stricken und/oder Flechten von Fasern zu einem Gewebe, Faservlies oder Filz.

4. Material nach mindestens einem der vorangehenden Ansprüche, wobei das Aggregatfasermaterial aus biokompatibler, wärmebeständiger Faser hergestellt ist.

5. Material nach Anspruch 4, wobei die biokompatible, wärmebeständige Faser eine anorganische oder Metallfaser ist.

6. Material nach Anspruch 5, wobei die anorganische Faser eine Kohlenstoffaser ist.

7. Material nach Anspruch 4, wobei die biokompatible, wärmebeständige Faser eine kohlenstoffbeschichtete Faser ist.

8. Material nach mindestens einem der vorangehenden Ansprüche, wobei das Aggregatfasermaterial Öffnungen oder freie Durchgänge mit einem Durchmesser von 20 bis 1000 um besitzt.

9. Material nach mindestens einem der vorangehenden Ansprüche, wobei das Kunststoffmaterial ein flexibles, biokompatibles Kunststoffmaterial ist.

10. Material nach Anspruch 9, wobei das flexible Kunststoffmaterial ein Fluorpolymer, Silikonharz, Polyvinylchlorid, Copolymer von Vinylidenchlorid, Polyethylen, Polypropylen, Polyester, Poly(hydroxymethylmethacrylate), Polyacrylamid, Polysulfon, Poly(N-vinylpyrrolidon) oder segmentiertes Polyurethan ist.

11. Material nach mindestens einem der vorangehenden Ansprüche, wobei an jeder Seite der Schicht aus Kunststoffmaterial eine entsprechende Schicht aus Aggregatfasermaterial gebunden ist.

12. Material nach mindestens einem der vorangehenden Ansprüche, wobei die Schichten verbunden sind mittels einer Schicht aus einem Klebemittel, die zwischen der Schicht aus Aggregatfasermaterial und der Schicht aus Kunststoffmaterial angeordnet ist.

13. Material nach Anspruch 12, wobei das Klebemittel ein Klebemittel ist, stammend von einem

7

Silikon, Copolymer aus Ethylen und Vinylacetat, Polyester, Nylon, Urethan-Elastomer, Vinylacetatharz oder Acrylharz.

14. Material nach mindestens einem der vorangehenden Ansprüche, wobei das Kunststoffmaterial ein elektrisch leitendes Material darin engearbeitet enthält.

15. Implantat oder künstliches Organ für den menschlichen Körper, wobei das Implantat oder künstliche Organ aus einem flexiblen, biokompatiblen, zusammengesetzten Material nach mindestens einem der vorangehenden Ansprüche hergestellt ist.

**Revendications**

1. Matériau composite biocompatible souple comprenant une couche de matériau de fibres agglomérées sur laquelle est fixée une couche de matière plastique, caractérisé en ce que les surfaces opposées de ces couches sont complètement liées ensemble et que cette couche de matériau de fibres agglomérées est arrangée pour que les cellules tissulaires y pénétrant.

2. Matériau suivant la revendication 1, caractérisé en ce que ce matériau de fibres agglomérées est un produit de traitement primaire.

3. Matériau suivant la revendication 2, caractérisé en ce que ce produit de traitement primaire a été obtenu par tricotage et/ou tressage de fibres en tissu, tissu non-tissé ou feutre.

4. Matériau suivant l'une quelconque des revendications 1 à 3, caractérisé en ce que ce matériau de fibres agglomérées est fait de fibres biocompatibles résistant à la chaleur.

5. Matériau suivant la revendication 4, caractérisé en ce que cette fibre biocompatible résistant à la chaleur est une fibre inorganique ou métallique.

6. Matériau suivant la revendication 5, caractérisé en ce que cette fibre inorganique est une fibre de carbone.

7. Matériau suivant la revendication 4, caractérisé en ce que cette fibre biocompatible résistant à la chaleur est une fibre revêtue de carbone.

8. Matériau suivant l'une quelconque des revendications 1 à 7, caractérisé en ce que ce matériau de fibres agglomérées a des ouvertures ou des stomates libres de 20 à 1000 µm de diamètre.

9. Matériau suivant l'une quelconque des revendications 1 à 8, caractérisé en ce que cette matière plastique est une matière plastique biocompatible souple.

10. Matériau suivant la revendication 9, caractérisé en ce que cette matière plastique est un fluoropolymère, une résine silicone, un chlorure de polyvinyle, un copolymère de chlorure de vinylidène, un polyéthylène, un polypropylène, un polyester, un poly(méthacrylate d'hydroxyméthyle), un polyacrylamide, une polysulfone, une poly(N-vinylpyrrolidone) et un polyuréthane à blocs.

11. Matériau suivant l'une quelconque des revendications 1 à 10, caractérisé en ce qu'il comprend une telle couche de matière plastique sur chaque face de laquelle est fixée une couche respective de matériau de fibres agglomérées.

12. Matériau suivant l'une quelconque des revendications 1 à 11, caractérisé en ce que les couches sont liées ensemble au moyen d'une couche d'agent adhésif interposée entre cette couche de matériau de fibres agglomérées et cette couche de matière plastique.

13. Matériau suivant la revendication 12, caractérisé en ce qu'un tel agent adhésif est un agent adhésif dérivé d'un silicone, d'un copolymère d'éthylène et d'acétate de vinyle, d'un polyester, d'un nylon, d'un élastomère d'uréthane, d'une résine d'acétate de vinyle ou d'une résin acrylique.

14. Matériau suivant l'une quelconque des revendications 1 à 13, caractérisé en ce qu'une matière électroconductrice est incorporée dans cette matière plastique.

15. Implant ou organe artificiel pour le corps humain, caractérisé en ce que cet implant ou cet organe artificiel est fait d'un matériau composite biocompatible souple suivant l'une quelconque des revendications précédentes.

# Fig. 1